# EUROPEAN PATENT APPLICATION

(11) **EP 4 335 842 A1**
(43) Date of publication of application: **13.03.2024**
(21) Application number: 22803748.7
(22) Date of filing: 24.04.2022
(51) Int. Cl.: C07D 295/24, B01D 1/22

(54) **PURIFICATION METHOD, SYSTEM AND DETECTION METHOD FOR N-METHYLMORPHOLINE N-OXIDE AND OBTAINED N-METHYLMORPHOLINE-N-OXIDE**

(30) Priority: 18.05.2021 CN 202110521394
(71) Applicant: B-FCTL Co., Ltd., Cangzhou, Hebei 061100 (CN)
(72) Inventor: LU, Wanli, Cangzhou, Hebei 061100 (CA); MA, Jie, Xi'an, Shaanxi 710018 (CN)
(74) Representative: Müller Schupfner & Partner Patent- und Rechtsanwaltspartnerschaft mbB
(86) International application number: PCT/CN2022/088743
(87) International publication number: WO 2022/242424

(57) **Abstract**

Disclosed are a purification method, system and detection method for N-methylmorpholine N-oxide (NMMO) and an obtained NMMO. The NMMO is derived from a crude product of NMMO prepared by reacting N-methylmorpholine with hydrogen peroxide. The mass concentration of NMMO in the crude NMMO product is 50-60%. The purification method comprises: cooling a crude NMMO product to -20°C-78°C for crystallization so as to obtain a crystal of NMMO. The NMMO purification method of the present invention has low costs. The obtained NMMO product has high purity and almost no "three wastes" are generated. Different from current NMMO purification process, the purification method of the present invention does not require an ion exchange resin; therefore, environmental problems such as large amounts of high-salt and high-COD wastewater and waste ion exchange resin brought about by the regeneration of an ion exchange resin are completely solved.

## Description

### FIELD

The invention relates to a purification method of N-methylmorpholine N-oxide, a purification system of N-methylmorpholine N-oxide, a detection method of N-methylmorpholine N-oxide and a N-methylmorpholine N-oxide obtained thereof.

### BACKGROUND

N-methylmorpholine N-oxide (methylmorpholine oxide, NMMO) is an excellent solvent with strong solubility for cellulose. At present, among the solvents that can dissolve cellulose, NMMO is the only solvent that can truly achieve industrialized production and has promising prospects. NMMO can dissolve cellulose well and cellulose solutions with good fiber forming and film forming properties can be obtained. Lyocell fibers obtained by spinning this solution have excellent fiber properties, and the NMMO solvent is easy to recover during the production process of lyocell fibers, with a recovery rate of over 99.5%. Lyocell fibers have significant advantages in environmental protection, energy consumption and fiber performance, and have a broad development prospect.

NMMO is prepared in industry by the reaction of hydrogen peroxide (H₂O₂) with N-methylmorpholine, and the NMMO crude product obtained by the reaction usually has a dark color. According to the applicant's (B-FCTL CO., LTD.) industrial preparation of NMMO in practice, the yield of the NMMO obtained by the reaction is over 99% and the total impurity mass in the NMMO crude product is less than 1%. The impurities contain a certain amount of unreacted N-methylmorpholine, morpholine, hydrogen peroxide and other impurities generated by side reactions, such as N-nitrosomorpholine, which is a strong carcinogen, organic peroxides and some unknown impurities. The impurities also contain a certain amount of metal cations, such as copper iron ions, and anions, such as phosphate and nitrate. The electrical conductivity of the NMMO crude product is generally above 500 µs/cm.

The quality standards for NMMO aqueous solution products with a mass concentration of 50% currently available for the production of Lyocell are that the electrical conductivity is less than 10 µs/cm, the content of copper ion is less than 2ppm, the content of iron ion is less than 2ppm, the content of unreacted hydrogen peroxide is less than 50ppm, the content of N-methylmorpholine is less than 100ppm, the content of morpholine is less than 200ppm, the content of N-nitrosomorpholine is less than 50ppb, and the products appearance is a colorless and transparent solution. For the production of Lyocell, the lower the content of these impurities in the NMMO aqueous solution products with a mass concentration of 50%, the better NMMO aqueous solution products will be. However, to further reducing the content of these impurities, it requires more complex and harsher purification steps and conditions than the current NMMO purification process, which will significantly increase the purification cost.

In addition, NMMO is an amine oxide, which will undergo the Cope elimination reaction at a certain temperature to form corresponding olefin compounds with double bonds at the terminal. Due to the presence of a large amount of hydrogen peroxide during the preparation process of NMMO, hydrogen peroxide will react with olefins to generate corresponding organic peroxides such as organic hydroperoxide, ether peroxide, and organic peroxide acid, thus the crude product of NMMO inevitably contain a certain amount of organic peroxide impurities.

The cellulose chain breakage in lyocell condition is mainly attributed to the radical of N-methylmorpholine produced by the radical decomposition reactions of NMMO. (Adorjan I., Potthast A., Rosenau T., Sixta H. and Kosma P.2005. Discoloration of cellulose solutions in N-methylmorpholine-N-oxide (Lyocell). Part 1: Studies on model compounds and pulps. Cellulose 12:51-57) (Rosenau T., Potthast A., Adorjan I., Hofinger A., Sixta H., Firgo H. and Kosma P. 2002. Cellulose solutions in N-methylmorpholime-N-oxide(NMMO)-Degradation processes and stabilizers. Cellulose 9: 283-291) . Peroxides, including inorganic peroxides, such as hydrogen peroxide, and organic peroxides, are good radical initiators that can decompose at a certain temperature to generate radicals, thereby inducing the occurrence of the radical decomposition reactions of NMMO, resulting in the breakage of cellulose chains and a decrease in the strength of lyocell fibers, and the final products of the radical decomposition reactions of NMMO are morpholine and formaldehyde, and there may be further reactions between morpholine and formaldehyde to generate the methylene morpholine carbocations. If the concentration of the methylene morpholine carbocations in the spinning solution formed by cellulose and NMMO is too high, a large number of intense exothermic autocatalytic decomposition reactions of NMMO will be induced, even resulting in an uncontrolled explosion (US 5556452). Therefore, peroxide is very harmful to Lyocell production. At present, more and more Lyocell manufacturers have realized the potential harm of organic peroxides in NMMO products to the production of Lyocell and have started to regulate the content of organic peroxide impurities in NMMO beside hydrogen peroxide. Although no further relevant standards having been introduced, the peroxide content of NMMO is required to be as low as possible.

During the production process, NMMO comes into contact with metal equipment, reactors, pipelines, and containers, resulting in that NMMO containing some metal ions such as copper and iron ions, while hydrogen peroxide also contains some metal and non-metallic ions. Copper and iron ions can catalyze the decomposition reaction of NMMO, causing the loss of NMMO. In the Lyocell condition, copper and iron ions in NMMO cellulose solution will also have complexation reaction with cellulose, causing cellulose degradation (Wu Cuiling, Li Xinping, Qin Shengli, and Wang Jianyong, "Study of new organic cellulose solvent: N-methylmorpholine N-oxide (NMMO)", Journal of Lanzhou University of Technology, 2005, 31 (2), 73-76). In addition, copper and iron ions will also have complexation reaction with propyl gallate (PG) added in the Lyocell production to produce a relatively dark color, which will affect the whiteness of the final lyocell fiber, and copper and iron ions will also catalyze the peroxide in the NMMO to oxidize cellulose, accelerate the degradation of cellulose, and catalyze Maillard reaction to produce color, and even catalyze the autocatalytic of NMMO to cause the decomposition reaction of radicals , release a large amount of heat, and explosion may occur in severe cases (such as CN 104801354 B and US 5556452). Hence, high amount of copper and iron ions pose a great threat to the production of NMMO, and the content of copper and iron ion in Lyocell grade NMMO products are required to be as low as possible.

At present, the industrial purification processes for NMMO are all multi-step purification processes, one of the steps using the method of ion-exchange resin to remove anions, cations, and some organic impurities, including chromophore impurities, from the NMMO crude product (CN 104801354 B), and other purification processes (such as X-ray irradiation and special adsorption resin) are used to remove N-nitrosomorpholine and other organic impurities. To obtain NMMO products with higher quality, special catalytic reduction treatment may be also required to further remove peroxides in the NMMO crude product (including hydrogen peroxide, organic hydroperoxide, ether peroxide, and organic peroxide acid). If only the ion-exchange resin is used to purify NMMO, non-ionic organic impurities, including peroxides, cannot be completely removed.

Exhausted ion-exchange resins need to be regenerated to be reused, wherein cation exchange resins typically use a 4-5% hydrochloric acid aqueous solution that is 4-6 times the volume of the resin for continuous rinsing and regeneration, while anion exchange resins use a 4-5% NaOH aqueous solution that is 4-6 times the volume of the resin for continuous rinsing and regeneration. After being neutralized by acid or alkali, these regeneration waste liquids do not only contain about 5% NaCl and other small amounts of metals and non-metallic ionic salts, but also contain other organic impurities and a small amount of NMMO adsorbed by the resin. Therefore, the COD content of the regeneration waste liquids is usually between 5000ppm-20000ppm. Generally, producing 1 ton of 50% NMMO aqueous solution product will have to generate 1-2 tons of the high-salinity wastewater with high COD. Due to its high salt content, this wastewater cannot be treated through the method of catalytic oxidation, as sodium and chloride ions can cause the deactivation of the Pt catalyst, and the wastewater cannot be directly incinerated through an organic waste liquid incinerator (TO), as high salt content does not only severely corrode the material of incinerator, but also seriously affects the operation of the incinerator due to a large amount of residual waste salt in the incinerator. Therefore, it is usually only possible to use Multiple-Effect Evaporation (MVR) to concentrate to obtain semisolid waste salts and separate the salts from the wastewater containing COD. However, this treatment method does not only consume high energy, but also produces the waste salts with high COD, which will be recognized as hazardous waste and will only be disposed of by qualified environmental protection companies at high cost. Moreover, the distilled water obtained from MVR usually has high COD content, and it must be treated through catalytic oxidation or direct incineration. In addition, ion-exchange resins and special adsorption resins capable of absorbing organic impurities have high prices and a lifespan of only 1-2 years, and spent resins are classified as hazardous waste according to the national hazardous waste classification list and must be disposed of by qualified environmental protection companies with high costs. At the same time, due to the adsorption of NMMO by ion exchange resins and special resins that adsorb organic impurities, the yield of NMMO is reduced. Therefore, the current industrial purification process for preparing Lyocell grade NMMO doesn't only face serious environmental issues such as exhaust gas, waste water, and solid waste, but also has high energy consumption and purification costs.

"Study of new organic cellulose solvent: N-methylmorpholine N-oxide (NMMO) "(Wu Cuiling, Li Xinping, Qin Shengli, and Wang Jianyong, Journal of Lanzhou University of Technology, 2005, 31 (2), 73-76) reveals that NMMO can combine with water molecules to form three types of hydrate crystals with different water contents. One NMMO molecule and one water molecule can form monohydrate crystal (NMMO • H₂O), a white crystal with melting point of 76-78 °C, NMMO content of 84.5% and a density of 1.28 g/cm³. Two NMMO molecules and five water molecules can also form disesquihydrate crystal (2NMMO • 5H₂O), a white crystal with melting point of 36-39 °C, NMMO content of 72.2% and a density of 1.33 g/cm³. One NMMO molecule and five water molecules can also form pentahydrate crystal (NMMO • 5H₂O), with melting point of -20 °C and NMMO content of 56.5%. NMMO • H₂O and NMMO • 2.5H₂O can be obtained by dehydration of NMMO aqueous solution. The article mainly investigated the cellulose solubility in different NMMO hydrate crystal and NMMO·H2O is used to dissolve cellulose by utilizing the characteristic of low melting point and its properties of good dissolution of cellulose.

### SUMMARY

A main object of the invention is to provide a purification method, system, and a detection method of N-methylmorpholine N-oxide, and a N-methylmorpholine N-oxide obtained thereof, so as to overcome the defects of high energy consumption, environmental pollution, low yield in the current NMMO purification process, and low purity of the N-methylmorpholine N-oxide obtained thereof in the prior art.

To achieve the above objects, the invention provides a purification method of NMMO, and the NMMO is derived from a NMMO crude product prepared by the reaction of N-methylmorpholine with hydrogen peroxide. The mass concentration of NMMO in the NMMO crude product is 50% ~ 60%, and the purification method includes: performing cooling crystallization to the NMMO crude product between -20 °C and 78 °C to obtain crystalline NMMO.

The purification method of NMMO in the invention, in one embodiment, the purification method further includes concentrating the NMMO crude product so that the mass concentration of NMMO in the NMMO crude product is 56.5% ~ 84.5%, and then the cooling crystallization is performed.

The purification method of NMMO in the invention, in one embodiment, seed crystals are added during the cooling crystallization process, and the amount of the seed crystals added is 0.1% ~ 1.0% of the weight of the NMMO crude product.

The purification method of NMMO in the invention, in one embodiment, after the concentration, when the mass concentration of NMMO in the NMMO crude product is 56.5% ~ 72.2%, the temperature of the cooling crystallization is -20°C ~ 39 °C; after the concentration, when the mass concentration of NMMO in the NMMO crude product is greater than 72.2% and less than or equal to 84.5%, the temperature of the cooling crystallization is greater than 39°C and less than or equal to 78 °C.

The purification method of NMMO in the invention, in one embodiment, after the concentration, when the mass concentration of NMMO in the NMMO crude product is 66% ~ 71%, the temperature of the cooling crystallization is 25°C ~ 35°C.

The purification method of NMMO in the invention, in one embodiment, during the cooling crystallization process, a cooling rate is 1 ~ 2°C/ hour from an initial appearance of crystals to 30°C; the cooling rate is 3 ~ 4°C/ hour between -20°C ~ 30°C.

The purification method of NMMO in the invention, in one embodiment, the cooling crystallization includes at least a primary cooling crystallization and a secondary cooling crystallization, the crystalline N-methylmorpholine N-oxide and a crystal mother solution are obtained by the primary cooling crystallization, and the crystal mother solution is first concentrated and then subjected to the secondary cooling crystallization.

To achieve the above objects, the invention further provides a N-methylmorpholine N-oxide obtained by the purification method of NMMO with a NMMO recovery rate of more than 97%. and the analysis of the N-methylmorpholine N-oxide using liquid chromatography shows undetectable content of organic impurities; the obtained NMMO is prepared into an aqueous solution with a mass concentration of 50%, the electrical conductivity of the aqueous solution is 0-10 µs/cm, and the content of hydrogen peroxide is 0.

To achieve the above objects, the invention further provides a purification method of NMMO, and the purification method does not use ion-exchange resin.

To achieve the above objects, the invention further provides a purification system of NMMO, and the purification system is provided for purification of NMMO from a NMMO crude product prepared by the reaction of N-methylmorpholine with hydrogen peroxide, and the purification system includes:
a crystallization device configured to crystallization process of the NMMO crude product;
a control device connected to the crystallization device to control crystallization conditions in the crystallization devices.

The purification system of NMMO in the invention, in one embodiment, the purification system further includes a concentration device connected to the crystallization device and the control device, so that the NMMO crude product is first concentrated in the concentration device and then input into the crystallization device; the control device controls concentration conditions of the concentration device.

To achieve the above objects, the invention further provides a detection method of NMMO, and the NMMO is obtained by cooling crystallization of a NMMO crude product prepared by the reaction of N-methylmorpholine with hydrogen peroxide; the detection method includes an analytical method to detect the residual rates of impurities in a crystalline NMMO, which includes following steps:
Step 1: performing the liquid chromatography analysis of the NMMO crude product with detection wavelength of 200nm~220nm or 200nm~210nm, and performing area integrals of all impurity peaks in the obtained chromatogram to obtain the sum of area integrals of all impurity peaks, which is recorded as a first sum of area integrals of impurity peaks;
Step 2: performing the liquid chromatography analysis of the NMMO with the same detection conditions as in step 1, and performing area integrals of all impurity peaks in the obtained chromatogram to obtain the sum of area integrals of all impurity peaks, which is recorded as a second sum of area integrals of impurity peaks;
   a first residual rate of crystal impurity = the second sum of area integrals of impurity peaks ÷ the first sum of area integrals of impurity peaks.

The detection method of NMMO in the invention, in one embodiment, the analytical method to detect the residual rates of impurities in the crystalline NMMO further includes following steps:
Step 3: performing the liquid chromatography analysis of the NMMO crude product with detection wavelength of 230nm-240nm, and performing area integrals of all impurity peaks in the obtained chromatogram to obtain the sum of area integrals of all impurity peaks, which is recorded as a third sum of area integrals of impurity peaks;
Step 4: performing the liquid chromatography analysis of the NMMO with the same detection conditions as in step 3, and performing area integrals of all impurity peaks in the obtained chromatogram to obtain the sum of area integrals of all impurity peaks, which is recorded as a fourth sum of area integrals of impurity peaks;
   a second residual rate of crystal impurity = the fourth sum of area integrals of impurity peaks ÷ the third sum of area integrals of impurity peaks.

The detection method of NMMO in the invention, in one embodiment, the detection method further includes an analytical method to detect the content of N-methylmorpholine and morpholine; the analytical method to detect the content of N-methylmorpholine and morpholine is: performing the liquid chromatography analysis of the NMMO with detection wavelength of 200nm~210nm and a detection time greater than or equal to 30 minutes.

The detection method of NMMO in the invention, in one embodiment, the detection method further includes an analytical method to detect the content of N-nitrosomorpholine; the analytical method to detect the content of N-nitrosomorpholine is: performing the liquid chromatography analysis of the NMMO with detection wavelength of 230nm~240nm and a detection time greater than or equal to 30 minutes.

The detection method of NMMO in the invention, in one embodiment, the detection method further includes an analytical method to detect at least one of hydrogen peroxide content, electrical conductivity, iron ion content, and copper ion content; the analytical method to detect the hydrogen peroxide content is performed in accordance with GB 5009.226-2016; the analytical method to detect the iron ion content or the copper ion content is performed by inductively coupled plasma optical emission spectroscopy (ICP-OES).

### The invention has the following beneficial effects:

The invention provides an efficient, low-cost, and green method for purifying NMMO, which mainly includes crystallization of the crude product; the crystallization can be run at room temperature (25-35 °C) and the energy consumption is low; the recovery rate of NMMO is above 97% and when the purified NMMO is diluted to 50% NMMO aqueous solution, the electrical conductivity of the NMMO aqueous solution is less than 10 µs/cm and is even as low as about 1 µs/cm, and the content of copper and iron ions are all less than 1 ppb, while HPLC cannot detect the presence of organic impurities such as N-nitrosomorpholine, for example, results of HPLC detection show that the total content of impurities does not exceed 2 ppm (i.e. the purity of NMMO > 99.999%), and the hydrogen peroxide content is also lower than the minimum detectable limit of the national standard GB 5009226-2016. The product quality meets or exceeds the NMMO product quality standards required by Lyocell production. Moreover, the NMMO purification method of the invention does not use ion-exchange resin, which completely solved the problem of environmental pollution faced in the current purification process using ion-exchange resin, in which large amount of wastewater with high concentration of salt and COD and spent ion exchange resin will have to be produced.

### BRIEF DESCRIPTION OF THE DRAWINGS

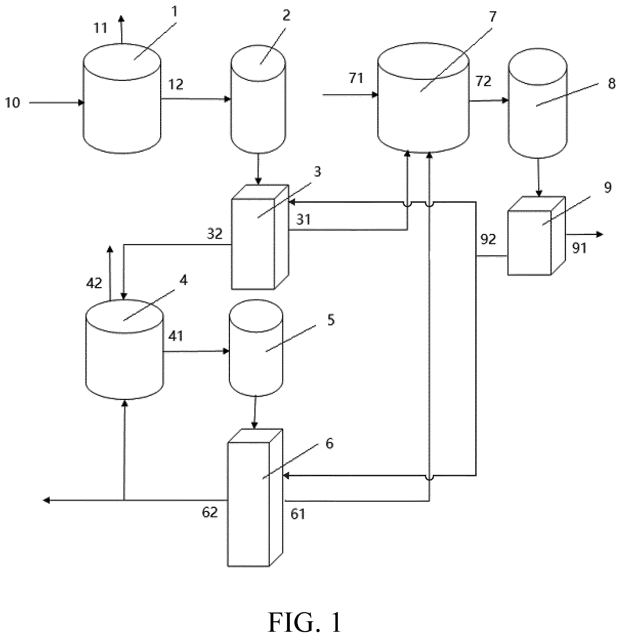

Figure 1 is a schematic diagram of a purification system of NMMO according to one embodiment of the invention.

### Reference numerals:

1 first concentration device
10 NMMO crude product
11 condensate
12 first concentrated solution
2 first crystallization device
3 first crystallization separation device
31 crystals from the first crystallization
32 mother solution of the first crystallization
4 second concentration device
41 second concentrated solution
42 condensate
5 second crystallization device
6 second crystallization separation device
61 crystals from the second crystallization
62 mother solution of the second crystallization
7 dissolution device
71 water
72 crystal solution
8 recrystallization device
9 recrystallization separation device
91 recrystallization crystals
92 recrystallization mother solution

### DETAILED DESCRIPTION

Embodiments of the invention are described in detail as follows. The embodiments are implemented under the premise of technical solutions of the invention and detailed implementation processes are provided, but the protection scope of the invention is not limited to the embodiments. Experimental methods for which no specific conditions are specified in the embodiments are generally based on conventional conditions. Unless otherwise specified, content refers to mass content and % refers to mass % in the invention.

The invention discloses a purification method of NMMO, which is used to obtain high purity N-methylmorpholine N-oxide from a NMMO crude product prepared by the reaction of N-methylmorpholine with hydrogen peroxide. The purification method of the invention does not use ion-exchange resin, so it can overcome the problems of environmental pollution and high energy consumption caused by using ion-exchange resin during purification. The purification method of the invention has characteristics of high efficiency, low energy consumption, low cost, green environmental protection, high yield and high purity of NMMO.

In one embodiment, the invention provides a purification method of NMMO from the NMMO crude product prepared by the reaction of N-methylmorpholine with hydrogen peroxide. The mass concentration of the NMMO in the NMMO crude product is 50% to 60%. The purification method includes: performing cooling crystallization to the NMMO crude product between -20 °C and 78 °C to obtain crystalline NMMO.

Crystallization purification method is performed by cooling down the temperature and taking advantage of the difference in solubility of each component of the mixture in a solvent, such that the concentration of the component to be crystallized and purified in the mother solution achieves the supersaturation and forming a supersaturated solution. The component to be crystallized and purified is precipitated in the supersaturated solution by forming crystals, while impurity components with a relatively low content is left in the mother solution, so as to achieve the effect of separation and purification. The main factors affecting the purity of crystals include adsorption of the mother solution on the crystals surface and embeddedness of the mother solution inside the crystals. In the invention, the crystalline NMMO is rinsed with pure saturated NMMO aqueous solution to remove the adsorbed mother solution on crystals surface, and crystallization process conditions are improved, or recrystallization is applied to improve the embeddedness problem of the mother solution in the crystals.

Due to transportation problems of pure NMMO in industry, and downstream industry using NMMO usually use NMMO aqueous solution as raw material, the NMMO is usually prepared as an aqueous solution in the industry.

The invention takes advantage of the characteristics of crystalline NMMO hydrate and controls the cooling crystallization conditions to combine the NMMO with water at a specific proportion to form crystals, thereby achieving the goal of separating the NMMO from impurities in aqueous solutions. In other words, the invention obtains a NMMO hydrate solid through crystallization, achieving the purpose of purifying the NMMO, and then the NMMO hydrate solid is prepared into an aqueous solution of a required concentration to supply to downstream customers.

In industry, the color of the NMMO crude product prepared by the reaction of N-methylmorpholine with hydrogen peroxide is generally brownish yellow, and depending on the concentration of hydrogen peroxide used, the mass concentration of NMMO in the crude product is between 50% -60%. The invention does not specifically limit the reaction process between N-methylmorpholine and hydrogen peroxide, and conventional reaction conditions in the field are sufficient.

In one embodiment, a concentrated solution with a mass content of NMMO ranging from 56.5% to 84.5% is obtained from the NMMO crude product by concentrating, and then cooling crystallization of the concentrated solution is performed. In this way, the crystallization efficiency can be improved. In another embodiment, the method of concentration is evaporation, such as heating evaporation, reduced pressure evaporation, and etc.

In one embodiment, the invention concentrates the NMMO crude product. When the mass content of NMMO in the obtained concentrated solution is between 56.5% -72.2%, performing cooling crystallization to the concentrated solution at temperatures between -20 ~ 39 °C, so that the obtained crystalline NMMO is 2NMMO • 5H₂O (i.e. NMMO • 2.5H₂O); when the mass content of NMMO in the obtained concentrated solution is between 72.2% - 84.5% (excluding 72.2%), performing cooling crystallization to the concentrated solution at temperatures between 39-78 °C (excluding 39 °C), so that the obtained crystalline NMMO is NMMO • H₂O. In other words, the different concentrations of NMMO in the concentrated solution determine the different crystallization temperatures, and further resulting in different crystalline NMMO.

In one embodiment, consider the cost of industrial purification and operation convenience, the NMMO crude product with a mass concentration of 66%-71% is obtained by concentration, and the following cooling crystallization can be performed to the concentrated solution at temperature range between 25-35°C (the final crystallization temperature is 25°C and above). Under the optimal crystallization conditions, 2NMMO • 5H₂O crystal with high purity and a crystallization yield of more than 50% can be obtained after first crystallization.

In one embodiment, seed crystals can be added during crystallization process with an amount of 0.1% to 1.0% of weight of the NMMO crude product, or 0.1% to 1.0% of weight of the concentrated solution mentioned above. In another embodiment, the amount of seed crystals added is 0.1% to 0.3% of weight of the NMMO crude product, or 0.1% to 0.3% of weight of the concentrated solution mentioned above. In cooling crystallization, adding seed crystals to induce crystallization can effectively reduce the degree of supersaturation of NMMO in solution, and resulting in crystals with higher NMMO purity.

In one embodiment, the seed crystals added in the invention are small particles of broken crystals with high purity, which is crushed and have a particle size as uniform as possible, and the size of the particles ranging from 0.01 to 0.1mm while the amount of the seed crystals added is 0.1 to 1.0% of weight of the NMMO crude product. Seed crystals with relatively large particles and insufficient addition of the seed crystals may lead to a decrease in the purity of crystalline NMMO, and the obtained crystalline NMMO have an uneven distribution of particle size; excessive addition of the seed crystals may lead to relatively small particles of the obtained crystalline NMMO. In addition, the seed crystals should not be added too early, Otherwise, the temperature of the solution may be too high, resulting in the possibility to dissolve the seed crystals due to the concentration of NMMO hydrate in the solution lower than NMMO hydrate solubility. The significance of adding the seed crystals will be lost; on the other hand, if the seed crystals are added too late, the temperature of the solution may be too low and the degree of supersaturation of the solution may be too high, which will reduce the actual effect of adding the seed crystals. The timing of adding the seed crystals is generally determined by the NMMO content in the NMMO crude product or the concentrated solution, and the initial cooling temperature. In one embodiment, seed crystals are added when the solution is cooled to 30-37 °C.

In one embodiment, the seed crystals of the invention are soaked in a high-purity NMMO saturated aqueous solution for at least 1 hour before being added to the crystallization system, which can improve crystallization effect and ensure crystals precipitated in the crystallization system have a uniform size.

In one embodiment, during the cooling crystallization process of the invention, from initial appearance of crystals in the crystallization system till the temperature of the crystallization system is cooled down to 30°C, the cooling rate is controlled at 1-2 °C/hour, and after the temperature of the crystallization system drops below 30 °C (for example, at -20-30°C), the cooling rate can be controlled at 3-4 °C/hour. In this way, crystal purity can be guaranteed. This is because the cooling rate directly determines the crystallization rate in the cooling crystallization. When the crystallization system is in a relatively high temperature range, the solubility curve of NMMO hydrate varies greatly. Therefore, in the initial stage of crystallization after the crystals begin to appear, the cooling rate of the solution system should not be too fast, and the growth rate of the crystals should be limited, otherwise it will lead to a high degree of supersaturation of the solution and affect crystal purity.

In one embodiment, after the cooling crystallization system reaches the crystallization termination temperature, the crystallization solution is quickly passed through a Buchner funnel for vacuum filtration, and the crystal mother solution is discharged from the filter bottle. The crystals are rinsed with the NMMO crude product and a high-purity NMMO aqueous solution with a concentration of 59.5±0.5%, respectively to obtain the crystalline NMMO, which are weighed and analyzed for content.

The invention adopts first crystallization to obtain the crystalline NMMO with high purity. The crystalline NMMO obtained from the first crystallization are mixed with water to prepare an NMMO aqueous solution with a mass concentration of 50%, which is colorless and transparent, and the electrical conductivity can reach 4-10 µs/cm.

To obtain a NMMO with higher purity, the cooling crystallization of the invention includes at least two crystallization processes, such as a primary crystallization and a secondary crystallization. That is, crystals obtained by the primary crystallization are mixed with water to prepare a NMMO aqueous solution with a certain concentration for the secondary crystallization (recrystallization). In one embodiment, the cooling crystallization of the invention also includes tertiary crystallization, quaternary crystallization, etc.

The crystals obtained by the secondary crystallization of the invention are analyzed and detected by liquid chromatography, and organic impurities such as N-nitrosomorpholine, N-methylmorpholine, morpholine, peroxide, etc. are not detectable. The crystals are mixed with water to prepare a NMMO aqueous solution with a mass content of 50%, which has an electrical conductivity of 3-6 µs/cm, and even as low as about 1 µs/cm.

In another embodiment, the crystals obtained by the secondary crystallization are mixed with water to prepare an aqueous solution of NMMO with a mass concentration of 50%, and the electrical conductivity of the NMMO solution (the mass concentration of NMMO is 50%) is about 1 µs/cm; by using Thermo Fisher ICP-MS detection, copper and iron ions are barely detected (indicating that the content of copper and iron ions is less than 0.001 ppm); according to the national standard GB 5009.226-2016, the presence of hydrogen peroxide cannot be detected. Therefore, the aqueous solution of NMMO with a mass concentration of 50% prepared by the crystals after the above treatment can fully meet the quality requirements for NMMO in Lyocell production and is far higher than the quality requirements for NMMO in Lyocell production.

In one embodiment, to improve the crystallization yield of NMMO, the invention includes at least a second crystallization, that is performing the first crystallization of the NMMO crude product to obtain the crystals and a crystal mother solution. Evaporating the crystal mother solution produced by the first crystallization through vacuum heating till the mass concentration of NMMO in the crystal mother solution is 69-71% or 69-70.5%, and then performing cooling crystallization (second crystallization), which has same crystallization conditions as the above first crystallization and the crystallization yield is around 80-85%. The obtained crystals can go back to dissolve in the NMMO crude product, which can be recycled and used as raw material liquid of the first crystallization, so as to further improve crystal purity. To further improve the crystallization yield of NMMO, the crystal mother solution obtained from second crystallization can continue to be concentrated and crystallized. At this time, the mass of the crystal mother solution obtained by the second crystallization is very small, which is generally less than 3% of the mass of the initial NMMO crude product.

In one specific embodiment, the purification method of NMMO of the invention includes: concentrating the NMMO crude product produced by the reaction of N-methylmorpholine with hydrogen peroxide by heating evaporation under reduced pressure to that the mass content of NMMO is 56.5 to 84.5% and obtaining high-purity NMMO hydrate crystals through cooling crystallization, thereby without the need for ion-exchange resin to obtain Lyocell grade NMMO products. In the purification method, the cooling crystallization can be run at the temperature between -20-78 °C. Considering the cost and operational suitability of industrial purification, it is preferable to control the mass concentration of NMMO in the crude product at 66-71%. In this way, the cooling crystallization can be performed at 25-35°C, and crystal products with high purity and a crystallization yield of more than 50% can be obtained.

In one specific embodiment, the method of cooling crystallization in the invention is as follows: the NMMO crude product with a NMMO mass concentration of 66.5-70% at a temperature of about 40°C is cooled down to the temperature at which the seed crystals will not dissolve after being added, and then a small amount of seed crystals is added. The seed crystals are preferably broken crystals of NMMO • 2.5H₂O in the form of small particle, which are pre-soaked in a high-purity NMMO saturated solution . The broken crystals in the form of small particle are soaked in the NMMO saturated solution for at least 1 hour, and the amount of seed crystals added is 0-1% of the mass of the NMMO crude product, which is preferably 0.1-0.3%. After adding seed crystals, the NMMO crude product is slowly cooled by cooling. The cooling rate is controlled at 1 - 2°C/ hour from the temperature of the initial appearance of crystals to 30°C. When the temperature drops below 30 °C, the cooling rate can be appropriately accelerated to 3-4 °C/hour, and the crystallization termination temperature is 25 °C. In this way, crystals in the form of large particle with an even distribution of particle size can be obtained. After rapid vacuum filtration of the crystal solution, the filtered mother solution is collected and weighed. The crystals are first rinsed with the NMMO crude product and the amount of the rinsing liquid of the crude product is about 30% of the mass of the obtained crystals. Then the crystals are rinsed with a high-purity NMMO aqueous solution with a mass concentration of 59.5±0.5% and the amount of the rinsing liquid is about 30% of the mass of the crystals, thereby primary crystalline crystals are obtained and collected to be weighed and analyzed for crystal purity. An aqueous solution with NMMO mass concentration of 50% is prepared by adding water to the primary crystalline crystals, and the contents of various impurities, hydrogen peroxide, metal ions and the electrical conductivity of the solution are detected.

Then the secondary crystallization is performed, and the process conditions of the secondary crystallization are similar to those of the primary crystallization. The primary crystalline crystals are dissolved in water at 40 °C to prepare an aqueous solution with a NMMO mass concentration of 66-70%. The secondary crystallization is performed under the same process conditions, and secondary crystalline crystals are collected to be weighed and analyzed for crystal purity.

In one specific embodiment, the purification method of NMMO in the invention is as follows: the NMMO crude product prepared by the reaction of N-methylmorpholine with hydrogen peroxide is concentrated to that the mass concentration of NMMO is 56.5-84.5% by vacuum heating, and high-purity NMMO hydrate crystals are obtained by cooling crystallization. The crystals are then added to water to prepare an aqueous solution with a NMMO mass concentration of 50%, which is a high-purity NMMO aqueous solution product with an electrical conductivity of as low as about 1µs/cm, a copper and iron ion content of both less than 0.001ppm. Performing second or more crystallization of the residual crystal mother solution to maximize the recovery of the NMMO in the crystal mother solution. The second crystallization of the crystal mother solution can recover more than 97% of NMMO in the NMMO crude product, and more crystallization will be able to further improve the recovery rate of NMMO.

Therefore, the invention provides a method for preparing a NMMO that meets the Lyocell quality requirements by purifying the NMMO from the reaction crude product of the NMMO through a crystallization method. Different from existing NMMO purification methods, the invention does not use ion-exchange resins to remove metal and non-metal ions, as well as some organic impurities, including chromophore impurities. As a result, the method of the invention produces almost zero amount of wastewater with high concentration of salt and COD content. Also, spent ion-exchange resin and other hazardous waste are not produced. The product of NMMO aqueous solution produced by the method of the invention can not only meets or even exceeds current Lyocell's quality requirements of NMMO, but also has many advantages such as simple purification process, low purification cost, and high yield of NMMO. The invention is an efficient, simple, low-cost and green purification method for NMMO.

In one embodiment, the invention also provides a purification system of NMMO, which is used to purify NMMO from a NMMO crude product prepared by the reaction of N-methylmorpholine with hydrogen peroxide. The purification system can be used for purification of NMMO in industrial production, thereby achieving large-scale automation control. The purification system includes:
a crystallization device configured to crystallization process of the NMMO crude product;
a control device connected to the crystallization device to control crystallization conditions in the crystallization device.

In another embodiment, the purification system further includes a concentration device connected to the crystallization device and the control device, so that the NMMO crude product is concentrated in the concentration device and then input into the crystallization device; The control device controls concentration conditions of the concentration device.

In one embodiment, the purification system of NMMO of the invention is shown in Figure 1. A first concentration device 1 is connected to a first crystallization device 2, and the NMMO crude product 10 flows into the first concentration device 1 for concentration process. The first concentration device 1, for example, is an evaporation device. Condensate 11 flows out from the top of the first concentration device 1, and an obtained first concentrated solution 12 flows into the first crystallization device 2 for crystallization process. The crystallization conditions have been explained in detail above and no further elaboration is made here.

The first crystallization device 2 is connected to a first crystallization separation device 3, and a mixture obtained from the crystallization in the first-crystallization device 2 flows into the first crystallization separation device 3 for solid-liquid separation to obtain crystals from the first crystallization 31 and a mother solution of the first crystallization 32.

In another embodiment, the first crystallization separation device 3 is connected to a second concentration device 4 such that the mother solution of the first crystallization 32 flows into the second concentration device 4 for concentration process, and a second concentrated solution 41 and a condensate 42 are obtained; the second concentration device 4 is connected to a second crystallization device 5, and the second concentrated solution 41 flows into the second crystallization device 5 for second crystallization process. In this way, the yield of the NMMO can be improved.

The second crystallization device 5 is connected to a second crystallization separation device 6, and a mixture obtained from the crystallization in the second crystallization device 5 flows into the second crystallization separation device 6 for solid-liquid separation to obtain crystals from the second crystallization 61 and a mother solution of the second crystallization 62. In one embodiment, the second crystallization separation device 6 is also connected to the second concentration device 4, so that the mother solution of the second crystallization 62 can be recycled back to the second concentration device 4 for concentration process, and then go back to the crystallization process again to further improve the yield of the NMMO. Due to the low content of the NMMO in the mother solution of the second crystallization 62, the mother solution of the second crystallization 62 can be directly discharged outside.

In one embodiment, the purification system of NMMO of the invention further includes a dissolution device 7, which is connected to the first crystallization separation device 3 and the second crystallization separation device 6 respectively such that the crystals from the first crystallization 31 and the crystals from the second crystallization 61 are transported to the dissolution device 7, and are mixed with input water 71 to obtain a crystal solution 72.

The dissolution device 7 is connected to a recrystallization device 8, which is also connected to a recrystallization separation device 9. The crystal solution 72 is transported to the recrystallization device 8 for recrystallization process, which can further improve the purity of the NMMO. A mixture obtained from the crystallization in the recrystallization device 8 is transported to the recrystallization separation device 9 to obtain recrystallization crystals 91 and a recrystallization mother solution 92. The Sample of the recrystallization crystals 91 is analyzed with liquid chromatography and the analysis results show that various organic impurities such as N-nitrosomorpholine, N-methylmorpholine, morpholine, peroxide, etc. in the NMMO crude product are not detectable. The recrystallization crystals 91 are mixed with water to prepare an NMMO aqueous solution with a mass content of 50% and the electrical conductivity of 3-6 µs/cm.

In one embodiment, the recrystallization separation device 9 is also connected to the first crystallization separation device 3 and the second crystallization separation device 6 respectively such that the recrystallization mother solution 92 is used for washing the crystals from the first crystallization 31 and the crystals from the second crystallization 61, reducing the mother solution adsorbed on the surface of the crystals from the first crystallization 31 and the crystals from the second crystallization 61, and further removing impurities embedded in the crystals from the first crystallization 31 and the crystals from the second crystallization 61.

In one embodiment, the purification system of NMMO of the invention is used for industrial production. The purification system also includes a control device (not shown in the figure), which is electrically connected to one or more of the first concentration device 1, the first crystallization device 2, the first crystallization separation device 3, the second concentration device 4, the second crystallization device 5, the second crystallization separation device 6, the dissolution device 7, the recrystallization device 8, and the recrystallization separation device 9 respectively, to control process parameters of the crystallization process.

In one embodiment, the invention provides a detection method of NMMO and the NMMO is obtained by crystallization of a NMMO crude product prepared by the reaction of N-methylmorpholine with hydrogen peroxide. The detection method includes an analytical method to detect the residual rates of impurities in the crystalline NMMO, which includes following steps:
Step 1, performing the liquid chromatography analysis of the NMMO crude product with a detection wavelength of 200nm-220nm, for example 200nm-210nm, and performing area integrals of all impurity peaks in the obtained chromatogram to obtain the sum of area integrals of all impurity peaks, which is recorded as a first sum of area integrals of impurity peaks;
Step 2, performing the liquid chromatography analysis of the NMMO with the same detection conditions as in step 1, and performing area integrals of all impurity peaks in the obtained chromatogram to obtain the sum of area integrals of all impurity peaks, which is recorded as a second sum of area integrals of impurity peaks;
A first residual rate of crystal impurity = the second sum of area integrals of impurity peaks ÷ the first sum of area integrals of impurity peaks.

In one embodiment, the analytical method to detect the residual rates of the impurities in the crystalline NMMO further includes following steps:
Step 3, performing the liquid chromatography analysis of the NMMO crude product with a detection wavelength of 230nm-240nm, and performing area integrals of all impurity peaks in the obtained chromatogram to obtain the sum of area integrals of all impurity peaks, which is recorded as a third sum of area integrals of impurity peaks;
Step 4, performing the liquid chromatography analysis of the NMMO with the same detection conditions as in step 3, and performing area integrals of all impurity peaks in the obtained chromatogram-to obtain the sum of area integrals of all impurity peaks, which is recorded as a fourth sum of area integrals of impurity peaks;
A second residual rate of crystal impurity = the fourth sum of area integrals of impurity peaks ÷ the third sum of area integrals of impurity peaks.

In this way, the invention can detect residual rates of impurity of the obtained crystal in different aspects by the analytical method to detect the first residual rate of crystal impurity and the second residual rate of crystal impurity.

In one embodiment, the detection method of NMMO of the invention further includes an analytical method to detect the content of N-methylmorpholine and morpholine, an analytical method to detect the content of N-nitrosomorpholine, and an analytical method to detect at least one of hydrogen peroxide content, electrical conductivity, iron ion content, and copper ion content.

The analytical method to detect the content of N-methylmorpholine and morpholine is: performing the liquid chromatography analysis of the NMMO with a detection wavelength of 200nm-210nm, and a detection time greater than or equal to 30 minutes. The analytical method to detect the content of N-nitrosomorpholine is: performing the liquid chromatography analysis of the NMMO with a detection wavelength of 230nm-240nm, and a detection time greater than or equal to 30 minutes. In this way, the content of N-methylmorpholine and morpholine can be obtained from the obtained chromatogram. The analytical method to detect the content of hydrogen peroxide shall be performed in accordance with GB 5009.226-2016; the analytical method to detect the content of iron ion or copper ion is performed by an inductively coupled plasma optical emission spectroscopy. The analytical method to detect the electrical conductivity can be performed according to the conventional methods in the field, and the invention is not specifically limited.

In one specific embodiment, the analytical method to detect the content of N-methylmorpholine and morpholine is: liquid chromatography, and chromatographic column is: AichromBond-1, C18, 5 µm 4.6x150mm; mobile phase: 0.2% phosphate buffer; flow rate of mobile phase: 1 ml/min; column temperature: 35 °C; UV detection wavelength: 200nm; sample size: 20 µl; detection time: 30 minutes.

The analytical method to detect the content of N-nitrosomorpholine is: liquid chromatography, and chromatographic column is: AichromBond-1, C18, 5 µm 4.6x150mm; mobile phase: 0.2% phosphate buffer; flow rate of mobile phase: 1 ml/min; column temperature: 35 °C; UV detection wavelength: 235nm; sample size: 20 µl; detection time: 30 minutes.

The invention uses impurity residual rate to show the purity of the obtained crystalline NMMO. In one specific embodiment, an analytical method to detect the impurity residual rate is: adding water to the NMMO crude product and crystals separately to prepare aqueous solutions with a NMMO mass concentration of 50%. The prepared NMMO aqueous solutions are detected by liquid chromatography, and the UV detection wavelengths are 200nm, 220nm, and 235nm respectively (detections are performed at three wavelengths respectively).

The invention utilizes the above analytical methods to detect a NMMO crude product prepared by the reaction of N-methylmorpholine with hydrogen peroxide provided in the following embodiments, and the obtained results are as follows:
N-methylmorpholine content: 1130ppm;
Morpholine content: 133ppm;
Hydrogen peroxide content: 634ppm;
N-nitrosomorpholine: 655 ppb,
Electrical conductivity: 732 µs/cm;
Iron ion content: 11.8ppm;
Copper ion content: 4.2ppm;

In addition, liquid chromatography analysis shows that the NMMO crude product also contains other organic impurities, including a large amount of organic peroxides.

The technical solution of the invention is further explained by specific embodiments as follows.

### Embodiment 1

This embodiment mainly analyzes the impact of different crystallization yields of NMMO on the purity of crystalline NMMO.

Evaporating the NMMO crude product by vacuum heating such that the mass concentration of NMMO is concentrated to 63.5%, 65.2%, 66.5%, 67.9% and 69.2% respectively and then cooling crystallization is performed respectively. Concentrated solutions of the NMMO crude product of each concentration start to cool down at 37°C, and temperatures of the solutions with NMMO mass concentrations of 63.5%, 65.2%, 66.5%, 67.9%, and 69.2% are cooled down to 31.2 °C, 32.1 °C, 33.2 °C, 34.2 °C, and 35.1 °C respectively. Seed crystals pre-soaked in NMMO saturated solution are added and the amount of the seed crystals is 0.15% of the mass of the NMMO crude product. After the seed crystals are added, the cooling rate is controlled at 1-2°C/ hour until the solutions temperature drops to 30°C, and then the cooling rate is accelerated to 3-4°C/ hour. When the solutions temperature drops to 25°C, stop the crystallization. Separating the mother solution from the crystals by rapid vacuum filtration and the mother solution is collected. The crystals are first rinsed with the crude product, which has a NMMO mass concentration of 58.2%, and the rinsing amount is approximately 30% of the crystal mass. Then the crystals are further rinsed with a high-purity NMMO aqueous solution with NMMO mass concentration of 59% and the rinsing amount is approximately 30% of the crystal mass. The crystals are collected and weighed to calculate the crystallization yield, and the purity of crystals (impurity residual rate) is analyzed and detected.

The NMMO crude product and the obtained crystals are separately added with water to prepare aqueous solutions with a NMMO mass concentration of 50% for analysis and detection. The results are shown in Table 1.

**Table 1. Impact of different crystallization yields on crystal purity**

| Initial mass concentration of crystallization solution | Crystallization yield | Impurity residual rate (detection at UV wavelength of 200nm) | Impurity residual rate (detection at UV wavelength of 220nm) | Impurity residual rate (detection at UV wavelength of 235nm) | Electrical conductivity (µs/cm) |
|---|---|---|---|---|---|
| Crude product with a NMMO mass concentration of 50% | | 100% | 100% | 100% | 732 |
| 63.5% | 27% | 0.97% | 0.45% | 0.009% | 3.0 |
| 65.2% | 43% | 0.95% | 0.43% | 0.011% | 3.2 |
| 66.5% | 52% | 0.97% | 0.44% | 0.010% | 3.4 |
| 67.9% | 65% | 1.0% | 0.45% | 0.010% | 3.4 |
| 69.2% | 75% | 1.20% | 0.48% | 0.012% | 3.5 |

As shown in Table 1, crystallization can effectively remove various impurities in the NMMO crude product, and impurity content and electrical conductivity in the obtained crystals are significantly reduced. When the crystallization yield is less than or equal to 65%, the purity of crystalline NMMO basically remains unchanged as the crystallization yield increases, but if the crystallization yield is greater than 65%, the purity of the crystals slightly decreases as the crystallization yield increases.

### Embodiment 2

This embodiment mainly analyzes the impact of different cooling rates on the purity of crystalline NMMO. Evaporating the NMMO crude product by vacuum heating such that the mass concentration of NMMO is concentrated to 67.9%. When the temperature of the NMMO crude product drops to 34.2 °C, NMMO seed crystals pre-soaked in NMMO saturated solution are added and the amount of the seed crystals is 0.15% of the mass of the NMMO crude product. The NMMO crude product is divided into four portions and crystallized at the following different cooling rates. When the crystallization solutions are cooled down to 25 °C, performing vacuum filtration while it is hot and analyzing the crystals according to the method in Embodiment 1.

### Cooling rates:

Cooling rate 1: cooling rate is controlled at 1 °C/hour from 34.2 °C to 30 °C, and cooling rate is controlled at 4 °C/hour between 30-25 °C.

Cooling rate 2: cooling rate is controlled at 2 °C/hour from 34.2 °C to 30 °C, and cooling rate is controlled at 4 °C/hour between 30-25 °C.

Cooling rate 3: cooling rate is controlled at 2 °C/hour from 34.2 °C to 30 °C, and cooling rate is controlled at 2 °C/hour between 30-25 °C.

Cooling rate 4: cooling rate is controlled at 4 °C/hour from 34.2 °C to 30 °C, and cooling rate is controlled at 4 °C/hour between 30-25 °C.

The crystalline NMMO are added with water to prepare a NMMO aqueous solutions with mass concentration of 50% for analysis and detection. The results are shown in Table 2.

**Table 2. Impact of different cooling rates on the purity of crystalline NMMO**

| Cooling rate | Crystallization yield | Impurity residual rate (detection at UV wavelength of 200nm) | Impurity residual rate (detection at UV wavelength of 220nm) | Impurity residual rate (detection at UV wavelength of 235nm) | Electrical conductivity (µs/cm) |
|---|---|---|---|---|---|
| Cooling rate 1 | 65% | 0.96% | 0.44% | 0.09% | 3.4 |
| Cooling rate 2 | 64% | 1.01% | 0.45% | 0.09% | 3.5 |
| Cooling rate 3 | 65% | 1.00% | 0.45% | 0.10% | 3.5 |
| Cooling rate 4 | 66% | 1.51% | 0.66% | 0.20% | 4.2 |

As shown in Table 2, during the initial crystallization stage (from the temperature of initial appearance of crystals to 30 °C), the cooling rate should not be too fast, and it is best to control the cooling rate at 1-2°C/hour, otherwise the crystal purity will significantly decrease; during late stage of cooling crystallization, the cooling rate can be appropriately accelerated. The main reason after analysis is that the solubility of crystalline NMMO varies greatly with temperature in the high-temperature region. Therefore, too rapid cooling can lead to a high degree of supersaturation of the solution, resulting in that more mother solution is embedded in the crystal and the crystal purity is reduced; during late stage of crystallization, the crystallization temperature is relatively low, and the trend of solubility of crystalline NMMO varies with temperature slows down, so the cooling rate can be appropriately accelerated.

### Embodiment 3

This embodiment mainly studies the impact of the amount of seed crystals added on crystal particle size and crystal purity. Evaporating the NMMO crude product by vacuum heating such that the mass concentration of NMMO is concentrated to 67.9%. The concentrated NMMO crude product is divided into 6 portions. When the temperature of the NMMO crude products drops to 34.2 °C, NMMO seed crystals pre-soaked in NMMO saturated solution are added respectively, the amount of which is: 0 (no seed crystals added), 0.05%, 0.1%, 0.2%, 0.3%, and 0.6% (as a percentage of the mass of the NMMO crude product) respectively, and then crystallization and analysis is performed to the crystals according to the method in Embodiment 1.

The crystalline NMMO are added with water to prepare aqueous solutions with a NMMO mass concentration of 50% for analysis and detection. The results are shown in Table 3.

**Table 3. Impact of different amount of seed crystals added on crystal purity and particle size**

| Amount of seed crystals added | Crystallization yield | Impurity residual rate (detection at UV wavelength of 200nm) | Impurity residual rate (detection at UV wavelength of 220nm) | Impurity residual rate (detection at UV wavelength of235nm) | Electrical conductivity (µs/cm) | Crystal particle size, distribution of particle size |
|---|---|---|---|---|---|---|
| 0 | No crystallization (the temperature drops to 25°C) | | | | | |
| 0.05% | 66% | 1.08% | 0.49% | 0.11% | 4.0 | Uneven distribution of particle size |
| 0.1% | 65% | 0.97% | 0.46% | 0.097% | 3.5 | Large particles with uniform distribution |
| 0.2% | 66% | 0.98% | 0.44% | 0.098% | 3.4 | Large particles with uniform distribution |
| 0.3% | 65% | 0.97% | 0.45% | 0.098% | 3.5 | Relatively large particles with uniform distribution |
| 0.6% | 66% | 1.03% | 0.47% | 0.10% | 3.8 | Relatively small particles with |
| | | | | | | uniform distribution |

As shown in Table 3, the absence of seed crystals has a significant impact on the crystallization process, and when the temperature drops to 25 °C, there is still no obvious crystal formation. When the amount of seed crystals added is relatively small (0.05%), the distribution of particle size is uneven, indicating that adding insufficient amount of seed crystals leads to relatively high degree of supersaturation of solution system and small crystal nucleus is spontaneously formed, resulting in a relatively low crystal purity. By increasing the amount of seed crystals added, the distribution of crystal particles becomes uniform, and the crystal purity increases. The larger the amount of seed crystals added, the smaller the particles. If excessive amount of seed crystals (0.6%) is added, the resulting crystal particles will be too small, and the surface of the particles may be contaminated with more impurities from the mother solution, resulting in a decrease in the crystal purity. At the same time, the small particle size of the crystals may affect the filtration speed of the crystals. The optimal amount of seed crystals added is 0.1-0.3% of the mass of the NMMO crude product.

### Embodiment 4

This embodiment mainly studies the impact of twice crystallizations on the purity of crystalline NMMO.

Evaporating the NMMO crude product by vacuum heating such that the mass concentration of NMMO is concentrated to 69.2%. When the temperature reaches 35.1 °C, seed crystals pre-soaked in NMMO saturated solution are added, and the amount of the seed crystals is 0.15% of the mass of the NMMO crude product. After the seed crystals are added, the cooling rate is controlled at 1-2°C/ hour until the temperature drops to 30°C, and then the cooling rate is accelerated to 4°C/ hour. Stopping the crystallization when the temperature drops to 25°C and performing rapid vacuum filtration while it is hot to separate the mother solution from the crystals and collect the mother solution as the raw material of crystallization experiment in Embodiment 5. The crystals are first rinsed with a NMMO crude product with a mass concentration of 60%, and the rinsing amount is approximately 30% of the crystal mass. Then the crystals are further rinsed with a high-purity NMMO aqueous solution with a mass concentration of 59.5%, and the rinsing amount is approximately 30% of the crystal mass. The crystals (primary crystallization) are collected and weighed to calculate the crystallization yield, and the crystal purity (impurity residual rate) is analyzed and detected.

The crystals obtained from the above primary crystallization are added with water to prepare an NMMO aqueous solution with a mass concentration of 68.1%. The secondary crystallization is performed in accordance with the above crystallization process, in which seed crystals are added at 34.3 °C. The obtained crystals are rinsed with a high-purity NMMO aqueous solution with a mass concentration of 59.5% and the rinsing amount is 30% of the crystal mass. The crystals (the secondary crystallization) are collected and weighed to calculate the crystallization yield, and the crystal purity (impurity residual rate) is analyzed and detected.

The crystalline NMMO obtained from the primary and secondary crystallization are added with water respectively to prepare aqueous solutions with a NMMO mass concentration of 50% for analysis and detection. The results are shown in Table 4.

**Table 4. Impact of twice crystallizations on crystal purity**

| Numb er of crysta llizati ons | Crys talliz ation yield | Colo r | Hydrog en peroxid e content | Impurity residual rate (detectio n at UV wavelen gth of 200nm) | Impurity residual rate (detectio n at UV wavelen gth of 220nm) | Impurity residual rate (detectio n at UV wavelen gth of 235nm) | N-nitroso morpho line content | Electr ical condu ctivity µs/cm | Iron ion cont ent | Cop per ion cont ent |
|---|---|---|---|---|---|---|---|---|---|---|
| prima ry crysta llizati on | 76% | Colo rless and trans pare nt | undetec table | 0.99% | 0.45% | 0.099% | 2.8 ppb | 3.6 | 2.0 ppb | 3.7 ppb |
| secon dary crysta llizati on | 67% | Colo rless and trans pare nt | undetec table | 0.02% | 0.01% | No obvious impurity peak | undetec table | 0.9 | unde tecta ble | 0.5 ppb |

Note: the above words " undetectable ", "not detected", or "no obvious impurity peak" indicate that the content of the substance to be detected is lower than the minimum detectable limit.

As shown in Table 4, the secondary crystallization can further purify NMMO, and significantly reduce the content of various organic impurities. In the aqueous solution with a concentration of 50% prepared by adding water to the crystalline NMMO, the electrical conductivity is reduced to 0.9 µs/cm, both copper and iron ions are less than 1 ppb, and the pH value is 11.4.

As the impurity content in the NMMO crude product used for crystallization is less than 1% and the results in Table 4 show that the crystals obtained from the twice crystallizations are analyzed with HPLC to have the impurity content of 0.02% of the impurity content in the NMMO crude product (at the wavelengths of UV detection: 200nm, 220nm, and 235nm). Therefore, the total impurity content in the crystals obtained from the twice crystallizations does not exceed 2ppm.

### Embodiment 5

This embodiment mainly studies the results of recovering NMMO from the residual mother solution of the primary crystallization through multiple crystallizations. The mother solution separated from the primary crystallization in Embodiment 4 is concentrated by heating evaporation under reduced pressure to that the mass concentration of NMMO is 70.1%. Crystallization is performed in accordance with the crystallization process conditions in Embodiment 4 (seed crystals are added when the temperature of the mother solution is 35.2 °C), and the obtained crystals are added with water to prepare an aqueous solution with a NMMO mass concentration of 50%. Then, analysis and detection are performed, and the results are shown in Table 5.

**Table 5. Results of recrystallization of primary crystallization mother solution**

| | Crystallizatio n yield | Impurity residual rate (detection at UV wavelengt h of 200nm) | Impurity residual rate (detection at UV wavelengt h of 220nm) | Impurity residual rate (detection at UV wavelengt h of 235nm) | N-nitrosomorpholin e content | Electrical conductivit y (µs/cm) |
|---|---|---|---|---|---|---|
| The mother solution of the primary crystallizati on concentrated to 70.1% of NMMO mass concentratio n | 84% | 2.45% | 1.01% | 0.23% | 35ppb | 5.1 |

As shown in Table 5, the primary crystallization mother solution is concentrated again and crystallized. When the crystallization yield reaches 84%, the purity of the obtained crystals is still very high. The total amount of the obtained crystalline NMMO from two crystallizations is equivalent to recycling 96% of the NMMO mass in the NMMO crude product. To further improve the yield of NNMO crystallization, concentration and crystallization of the mother solution of the second crystallization can be needed, or the improvement of total crystallization yield of the second crystallization can be needed. The crystals obtained from multiple crystallizations of the mother solution can be returned to be dissolved in the NMMO crude product as raw materials for the primary crystallization.

### Industrial Application

In summary, the invention provides an efficient, low-cost, and green method for purification and preparation of high-purity NMMO aqueous solution, including cooling crystallization process, and crystallization temperature closes to room temperature (25-35 °C). Reaction crude products of NMMO are purified by the purification method provided in the invention, and the NMMO recovery rate is above 96%. The electrical conductivity of the NMMO aqueous solution with a mass concentration of 50% which is prepared with the obtained crystalline NMMO by the crystallization of the invention is about 1µs/cm, and the content of copper and iron ions of the NMMO aqueous solution both are less than 1 ppb; results of HPLC detection show that the total content of organic impurities does not exceed 2 ppm (i.e. the purity of NMMO >99.999%); the presence of N-nitrosomorpholine is almost undetectable by HPLC, and the content of hydrogen peroxide is also lower than the minimum detectable limit of the national standard GB 5009226-2016. The product quality meets or exceeds the NMMO product quality standard required for Lyocell production. Furthermore, the NMMO purification method of the invention does not use ion-exchange resin. Therefore, it has completely solved environmental problems caused by the wastewater with high concentration of salt and COD and spent ion-exchange resin faced in the current purification process using ion-exchange resin.

The NMMO purification method provided in the invention is also applicable to other fields that involve the use and need of NMMO purification, such as the NMMO purification process of coagulating bath in Lyocell production, the recovery and purification process of the waste liquid of the semiconductor chip etching agent containing NMMO, and the recovery and purification process of the waste liquid of electronic cleaning solution containing NMMO.

Of course, the invention can still have various other embodiments, and without departing from the spirit and essence of the invention, the person skilled in the art can make various corresponding modifications and variations according to the invention, but these corresponding modifications and variations should belong to the protection scope of the claims of the invention.

## Claims

1. A purification method of N-methylmorpholine N-oxide, which is used for a mixture containing N-methylmorpholine N-oxide, the purification method includes: performing cooling crystallization to the mixture between -20 °C and 78 °C to obtain crystalline N-methylmorpholine N-oxide.

2. The purification method of N-methylmorpholine N-oxide according to claim 1, wherein the mixture is a crude product of N-methylmorpholine N-oxide prepared by the reaction of N-methylmorpholine with hydrogen peroxide, and the mass concentration of N-methylmorpholine N-oxide in the N-methylmorpholine N-oxide crude product is 50% ~ 60%.

3. The purification method of N-methylmorpholine N-oxide according to claim 2, wherein the purification method further includes concentrating the N-methylmorpholine N-oxide crude product so that the mass concentration of N-methylmorpholine N-oxide in the N-methylmorpholine N-oxide crude product is 56.5% ~ 84.5%, and then the cooling crystallization is performed.

4. The purification method of N-methylmorpholine N-oxide according to claim 2, wherein seed crystals are added during the cooling crystallization process, and the amount of the seed crystals added is 0.1% ~ 1.0% of the weight of the N-methylmorpholine N-oxide crude product.

5. The purification method of N-methylmorpholine N-oxide according to claim 3, wherein after the concentration, when the mass concentration of N-methylmorpholine N-oxide in the N-methylmorpholine N-oxide crude product is 56.5% ~ 72.2%, the temperature of the cooling crystallization is -20°C ~ 39 °C; after the concentration, when the mass concentration of N-methylmorpholine N-oxide in the N-methylmorpholine N-oxide crude product is greater than 72.2% and less than or equal to 84.5%, the temperature of the cooling crystallization is greater than 39 °C and less than or equal to 78 °C.

6. The purification method of N-methylmorpholine N-oxide according to claim 5, wherein after the concentration, when the mass concentration of N-methylmorpholine N-oxide in the N-methylmorpholine N-oxide crude product is 66% ~ 71%, the temperature of the cooling crystallization is 25°C ~ 35°C.

7. The purification method of N-methylmorpholine N-oxide according to claim 1, wherein during the cooling crystallization process, a cooling rate is 1 ~ 2°C/ hour from an initial appearance of crystals to 30°C; the cooling rate is 3 ~ 4°C/ hour between -20°C ~ 30°C.

8. The purification method of N-methylmorpholine N-oxide according to claim 1, wherein the cooling crystallization includes at least a primary cooling crystallization and a secondary cooling crystallization, the crystalline N-methylmorpholine N-oxide and a crystal mother solution are obtained by the primary cooling crystallization, and the crystal mother solution is first concentrated and then subjected to the secondary cooling crystallization.

9. A N-methylmorpholine N-oxide obtained by the purification method of N-methylmorpholine N-oxide according to claim 1-8, wherein the analysis of the obtained N-methylmorpholine N-oxide using liquid chromatography shows undetectable content of organic impurities; the obtained N-methylmorpholine N-oxide is prepared into an aqueous solution with a mass concentration of 50%, the electrical conductivity of the aqueous solution is 0-10 µs/cm, and the content of hydrogen peroxide is 0.

10. Application of the N-methylmorpholine N-oxide according to claim 9 in cellulose.

11. A purification method of N-methylmorpholine N-oxide, wherein the purification method does not use ion-exchange resin.

12. A preparation method of N-methylmorpholine N-oxide, including following steps:
Step 1, N-methylmorpholine reacts with hydrogen peroxide to produce a N-methylmorpholine N-oxide crude product;
Step 2, performing cooling crystallization to the N-methylmorpholine N-oxide crude product between -20 °C and 78 °C to obtain crystalline N-methylmorpholine N-oxide.

13. A purification system of N-methylmorpholine N-oxide, wherein the purification system is provided for purification of N-methylmorpholine N-oxide from a mixture containing N-methylmorpholine N-oxide, and the purification system includes:
a crystallization device configured to crystallization process of the mixture;
a control device connected to the crystallization device to control crystallization conditions in the crystallization device.

14. The purification system of N-methylmorpholine N-oxide according to claim 13, wherein the purification system further includes a concentration device connected to the crystallization device and the control device, so that the mixture is first concentrated in the concentration device and then input into the crystallization device; the control device controls concentration conditions of the concentration device.

15. The purification system of N-methylmorpholine N-oxide according to claim 13, wherein the mixture is a N-methylmorpholine N-oxide crude product prepared by the reaction of N-methylmorpholine with hydrogen peroxide; the purification system is used for purification of N-methylmorpholine N-oxide in industrial production.

16. A detection method of N-methylmorpholine N-oxide, wherein the N-methylmorpholine N-oxide is obtained by cooling crystallization of a N-methylmorpholine N-oxide crude product prepared by the reaction ofN-methylmorpholine with hydrogen peroxide, and the detection method includes an analytical method to detect the residual rates of impurities in a crystalline N-methylmorpholine N-oxide, which includes following steps:
Step 1, performing the liquid chromatography analysis of the N-methylmorpholine N-oxide crude product with a detection wavelength of 200nm-220nm, and performing area integrals of all impurity peaks in the obtained chromatogram to obtain the sum of area integrals of all impurity peaks, which is recorded as a first sum of area integrals of impurity peaks;
Step 2, performing the liquid chromatography analysis of the N-methylmorpholine N-oxide with the same detection conditions as in step 1, and performing area integrals of all impurity peaks in the obtained chromatogram to obtain the sum of area integrals of all impurity peaks, which is recorded as a second sum of area integrals of impurity peaks;
a first residual rate of crystal impurity = the second sum of area integrals of impurity peaks ÷ the first sum of area integrals of impurity peaks.

17. The detection method of N-methylmorpholine N-oxide according to claim 16, wherein the analytical method to detect the residual rates of impurities in the crystalline N-methylmorpholine N-oxide further includes following steps:
Step 3, performing the liquid chromatography analysis of the N-methylmorpholine N-oxide crude product with a detection wavelength of 230nm-240nm, and performing area integrals of all impurity peaks in the obtained chromatogram to obtain the sum of area integrals of all impurity peaks, which is recorded as a third sum of area integrals of impurity peaks;
Step 4, performing the liquid chromatography analysis of the N-methylmorpholine N-oxide with same detection conditions as in step 3, and performing area integrals of all impurity peaks in the obtained chromatogram to obtain the sum of area integrals of all impurity peaks, which is recorded as a fourth sum of area integrals of impurity peaks;
a second residual rate of crystal impurity = the fourth sum of area integrals of impurity peaks ÷ the third sum of area integrals of impurity peaks.

18. The detection method of N-methylmorpholine N-oxide according to claim 16, wherein the detection method further includes an analytical method to detect the content of N-methylmorpholine and morpholine; the analytical method to detect the content of N-methylmorpholine and morpholine is: performing the liquid chromatography analysis of the N-methylmorpholine N-oxide with a detection wavelength of 200nm-210nm, and a detection time greater than or equal to 30 minutes.

19. The detection method of N-methylmorpholine N-oxide according to claim 16, wherein the detection method further includes an analytical method to detect the content of N-nitrosomorpholine; the analytical method to detect the content of N-nitrosomorpholine is: performing the liquid chromatography analysis of the N-methylmorpholine N-oxide with a detection wavelength of 230nm-240nm, and a detection time greater than or equal to 30 minutes.

20. The detection method of N-methylmorpholine N-oxide according to claim 16, wherein the detection method further includes an analytical method to detect at least one of hydrogen peroxide content, electrical conductivity, iron ion content, and copper ion content; the analytical method to detect the hydrogen peroxide content is performed in accordance with GB 5009.226-2016; the analytical method to detect the iron ion content or the copper ion content is performed by an inductively coupled plasma optical emission spectroscopy.
